# EUROPEAN PATENT SPECIFICATION

(11) **EP 1 941 849 B1**
(45) Date of publication and mention of the grant of the patent: **14.07.2010**
(21) Application number: 08005039.6
(22) Date of filing: 01.10.1997
(51) Int. Cl.: A61F 9/01, A61B 18/20

(54) **Apparatus for corneal laser surgery**
Einrichtung zur Hornhaut-Laserchirurgie
Dispositif pour chirurgie laser de la cornée

(30) Priority: 02.10.1996 US 725070
(43) Date of publication of application: 09.07.2008
(62) Divisional of application: 97945439.4
(73) Proprietor: AMO Development, LLC, Santa Ana, CA 92705 (US)
(72) Inventor: Tibor, Juhasz, Irvine, CA 92715-4038 (US)
(74) Representative: Grünecker, Kinkeldey, Stockmair & Schwanhäusser Anwaltssozietät

(56) References cited:
- WO-A-93/08878
- WO-A-94/09849
- US-A- 4 842 599
- US-A- 4 907 586

## Description

### FIELD OF THE INVENTION

The present invention pertains generally to ophthalmic surgery which is useful for correcting vision deficiencies. More particularly, the present invention pertains to apparatus which surgically correct the vision of a patient by removing portions of the stroma to reshape the cornea. The present invention is particularly, but not exclusively useful in a method for using a laser beam to photodisrupt corneal tissue to achieve access to a predetermined volume of stromal tissue which needs to be removed to correct the vision of the patient.

### BACKGROUND OF THE INVENTION

Vision impairment can occur for many reasons, and be the result of many causes. One, all too common, cause for vision impairment results from a defective condition of the eye which occurs when the refractive characteristics of the cornea do not cause parallel rays of light to focus on the retina. When the eye is at rest, and the rays of light focus in front of the retina, the condition is known as myopia (i.e. near-sightedness). On the other hand, when the rays of light focus behind the retina, the condition is known as hypermetropia or hyperopia (i.e. far-sightedness). Both myopic and hyperopic conditions result in varying degrees of vision impairment and, as is well known, in most cases the conditions are correctable.

Spectacles or eyeglasses are commonly used to correct myopic or hyperopic conditions. For various reasons, however, many persons who suffer with these conditions prefer not to wear eyeglasses. Fortunately for these individuals, it is known that surgical procedures can be employed which will reshape the cornea in ways that are effective in changing its refractive characteristics. For example, U.S. Patent NO. 4,665,913 which issued to L'Esperance for an invention entitled "Method for Ophthalmological Surgery", and U.S. Patent No. 4,669,466 which issued to L'Esperance for an invention entitled "Method and Apparatus for Analysis and correction of Abnormal. Refractive Errors of the Eye" both disclose a laser system which photoablates corneal tissue from the anterior surface of the eye. In a different manner, U.S. Patent No. 4,988,348 which issued to Bille for an invention entitled "Method for Reshaping the Comea", and which is assigned to the same assignee as the present invention, discloses a procedure whereby corneal tissue is first removed to correct vision, and then the newly created surface is smoothed.

WO-A-9409849 is considered to represent the closest prior art, and discloses computer controlled laser surgical apparatus adapted and programmed to excise a layer of corneal tissue by directing a focused laser beam along a path, the path defining the cut surface.

Rather than remove and reshape portions of the anterior portion of the eye to correct refractive defects, some procedures for reshaping the cornea have suggested intrastromal photoablation for removal of only stromal tissue. As an example of such a procedure, U.S. Patent No. 4,907,586, which issued to Bille et al. for an invention entitled "Method for Reshaping the Eye" discloses an intrastromal photodisruption technique for reshaping the cornea. Another example of a procedure which is intended to essentially remove only stromal tissue is the so-called "flap and zap" procedure. For this procedure, an anterior portion of the cornea is removed and a portion of the exposed stroma is then photoablated. The previously removed anterior portion of the cornea is then repositioned on the cornea to cover the photodisruption. This procedure, like the procedure disclosed in Bille et al. '586, has as its objective the removal of only stromal tissue with the consequent preservation of anterior corneal tissue. A significant downside for the "flap and zap" procedure, however, is the possibility that the previously removed anterior portion of the cornea may again become detached. While the intrastromal procedure disclosed by Bille et al. does not lead to this detachment problem it can, in some cases, require extensive laser photodisruption and be time consuming.

It is appreciated by the present invention that the "flap and zap" procedure can be made more effective and efficient if the "flap" that is created can somehow be repositioned in an interlocking relationship with the undisturbed corneal tissue. To accomplish this, the present invention recognizes that it would be desirable if, first, a "flap" with an interlockable configuration is created. The flap could then be lifted to expose the corneal tissue that is to be removed and, next, after the desired amount of corneal tissue is removed, the flap could be repositioned and interlocked with undisturbed corneal tissue to hold the "flap" in place during the healing process.

The use of laser systems for ophthalmic surgical procedures, such as for other procedures contemplated for the present invention, is particularly appropriate due to the extreme precision required when corneal tissue is to be removed. Specifically, depending on the diameter and the general shape of the tissue volume to be removed, it is known that the removal of a layer of stromal tissue which is only approximately ten microns thick will result in a one diopter change. More practically, by way of example, the removal of a lens shaped volume of tissue which is four millimeters in diameter and approximately fifty microns thick at its center will result in a refractive correction of approximately four diopters. In almost all cases, for precise vision corrections which can stay within a one diopter accuracy, the surgical procedure employed must be capable of removing corneal tissue having a thickness which is accurate to within less than ten microns. Furthermore, this degree of accuracy applies for any refractive correction regardless of the total amount of correction required.

It happens that the correction of myopia requires removal of a differently shaped volume of corneal tissue than does the correction of hyperopia. Also, the limits of potential correction are different. Specifically, for a myopic correction it is known that a lentoid or lens shaped volume of stromal tissue needs to be removed. At the present time, myopic corrections of up to approximately thirty diopters can be reasonably expected. On the other hand, corrections of hyperopic conditions can be made up to only about fifteen diopters. Furthermore, for a hyperopic correction a generally doughnut shaped volume of stromal tissue, rather than a lens or lentoid shaped volume, needs to be removed.

Anr object of the present invention is to provide apparatus for corneal laser surgery which creates an interlocking flap that can be lifted to remove a predetermined volume of tissue from the stroma and then repositioned in an interlocking relationship with undisturbed corneal tissue to hold the flap in place during subsequent healing.

### SUMMARY OF THE PREFERRED EMBODIMENTS

In accordance with the present invention, apparatus is provided as in claim 1 appended hereto.

As intended for the present invention, with the removal of the lentoid volume of stromal tissue, the cornea is appropriately reshaped to correct the particular vision defect of the patient.

As intended for the present invention, the laser system will incorporate a beam of sequential laser pulses. Further, it is contemplated that the duration of laser pulses in the beam will be in the nanosecond, picosecond or femtosecond ranges.

### BRIEF DESCRIPTION OF THE DRAWINGS

The novel features of this invention, as well as the invention itself, both as to its structure and its operation will be best understood from the accompanying drawings, taken in conjunction with the accompanying description, in which similar reference characters refer to similar parts, and in which:
Figure 1 is a perspective view of a patient being treated with the apparatus of the present invention;
Figure 2 is a perspective view of an eye;
Figure 3 is a cross sectional view of the cornea of the eye as seen along the line 3-3 in Figure 2 showing a representative portion of stromal tissue to be removed for the correction of myopia;
Figure 4 is a plan view of the cornea of the eye as seen in the direction of the line 4-4 in Figure 2 showing a representative path for movement of the laser beam focal point to prepare the portion of stromal tissue shown in Figure 3 for removal from the cornea;
Figure 5 is a cross sectional view of the cornea of an eye; and
Figure 6 is a plan view of the portion of the cornea shown in Figure 5 looking at the anterior surface thereof in a posterior direction.

### DESCRIPTION OF THE PREFERRED EMBODIMENT

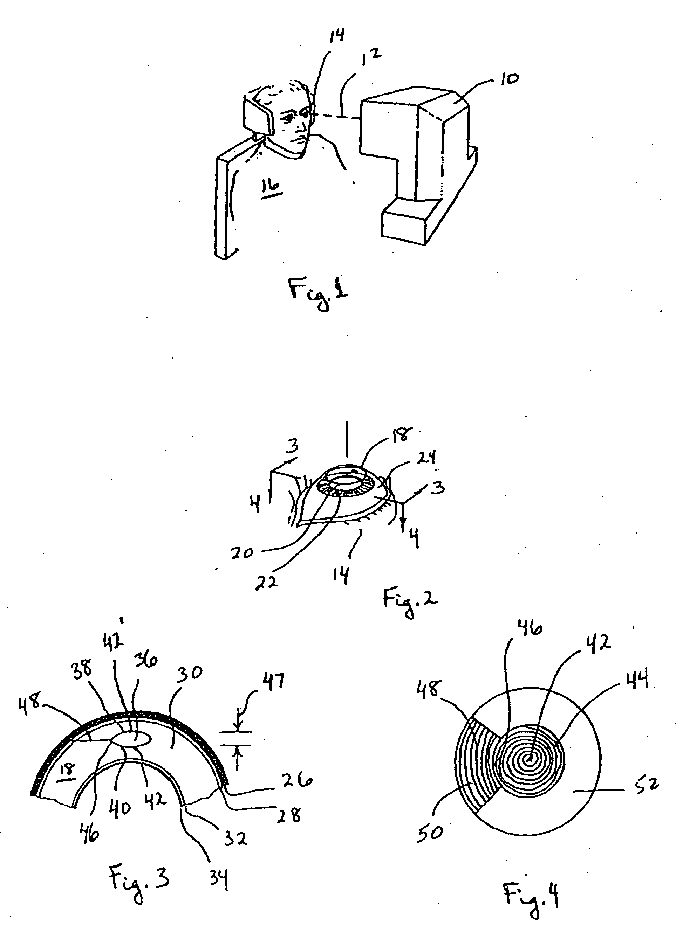

Referring initially to Figure 1, an apparatus 10 for generating a laser beam 12 is shown. Specifically, the laser beam 12 is shown being directed onto an eye 14 of a patient 16. For purposes of the present invention, the apparatus 10 is capable of generating a pulsed laser beam 12 having physical characteristics similar to those of the laser beams generated by a laser system as disclosed and claimed in U.S. Patent No.4,764,930, which is also assigned to the assignee of the present invention. Furthermore, the present invention contemplates the use of a pulsed laser beam 12 which has pulses with durations as long as a few nanoseconds or as short as only a few femtoseconds.

Figure 2 shows the anatomical structure of eye 14 and, specifically, that the cornea 18 is anterior to the pupil 20, the iris 22, and the sclera 24. Additionally, Figure 2 indicates that the optical axis 26 of eye 14 passes through the cornea 18. Consequently, the tissue of cornea 18 is transparent to visible light.

In Figure 3 it can be seen that the cornea 18 includes five anatomically definable layers of tissue. Going in a direction from anterior to posterior in Figure 3, the tissue layers of the cornea are: epithelium 26, Bowman's membrane 28, stroma 30, Decemet's membrane 32 and endothelium 34. Of these, the stroma 30 is of most importance for the present invention as it contains the only tissue which is to be removed for correction of the patient's vision.

As indicated above, the correction of a myopic condition can be accomplished by the removal of a predetermined volume of stromal tissue. As also indicated above, the particular volume of stromal tissue to be removed for the correction of myopia will depend on the amount of correction required and will be a lens or lentoid shaped volume. Such a lentoid volume 36 is shown in cross section in Figure 3. As shown, it is to also be appreciated that the lentoid volume 36 will be defined by an anterior surface 38 and a posterior surface 40. Together, the anterior surface 38 and the posterior surface 40 will completely enclose or encapsulate the lentoid volume 36 of stromal tissue 30 which is to be removed. To obtain the lens shape of the lentoid volume 36 it will be understood and further appreciated that, when considering lentoid volume 36 in a direction from anterior to posterior, the anterior surface 38 may be convex in shape and the posterior surface 40 may be concave in its shape.

It is to be appreciated that the actual shape for lentoid 36 may vary according to the needs and desires of the physician.

The creation of the anterior surface 38 and posterior surface 40 of lentoid volume 36 will, perhaps, be best appreciated with cross reference between Figure 3 and figure 4. in Figure 4, a predetermined start point 42 is shown, which is preferably on the posterior surface 40. The laser beam 12 is then focused initially on the predetermined start point 42 and, subsequently, the focal point of the laser beam 12 is moved according to computer programmed instructions along the spiral path 44. The spiral projection of the laser beam's focal point continues along spiral path 44 to create the concave posterior surface 40 until it reaches a point 46. Upon reaching the point 46 for the first time, the laser beam 12 is focused at a start point 42' on the anterior surface 38 of lentoid volume 36. The focal point of the laser beam 12 is then moved, again according to computer programmed instructions along a spiral path 42' to create the convex anterior surface 38 until the focal point again arrives at the point 46. With these actions the lentoid volume 36 is encapsulated and surrounded by photodisrupted tissue in the surfaces 38 and 40. For most applications the maximum distance 47 between the surfaces 38 and 40 will not exceed two hundred and fifty microns.

laser beam 12 is activated and the channel 48 is created over the remaining approximately ninety degrees of travel for the laser beam 12 focal point

As implied above, it may be preferable to generate a complete spiral path 50, rather than the partial spiral path 50 shown in Figure 4. To do this, laser beam 12 remains activated during photodisruption of stromal tissue during each complete 360° sweep of laser beam 12 along path 50. Thus, no space 52 is created and, instead, the spiral path 50 creates a layer of photodisrupted tissue. With this complete spiral path 50 pattern, it is subsequently possible to create an access channel 48 to the lentoid volume 36 from any direction. Additionally, the tissue of stroma 30 which is photodisrupted by each complete 360° sweep of laser beam 12 is symmetrically disposed around the lentoid volume 36. In some cases, this symmetrical disposition of photodisrupted tissue may be necessary in order to prevent a later development of irregular astigmatism.

Once the lentoid volume 36 of stromal tissue 30 has been created, as disclosed above. The computer controlled apparatus 10 of the present invention, can be used to create a section of comeal tissue which can be moved to expose, and thereby establish access to, the capsule volume 36 that is to be removed. For this particular procedure a layer 72 of corneal tissue is created which has an undercut region 74 that structurally interacts, or interlocks, with an intact overlap region 76 of the cornea. The idea here is to have the undercut region 74 interlock with the overlap region 76 so that the layer 72 does not unintentionally move in an anterior direction. For purposes of this disclosure, the anterior direction is to be considered as the general direction taken from the posterior surface 78 of cornea 18 (i.e. endothelium 34) toward the anterior surface 80 of cornea 18 (i.e. epithelium 26). Contrarily, the posterior direction is taken to be from the anterior surface 80 back toward the posterior surface 78.

For a description of how to create the layer 72, it is best to first identify a reference axis 82 from which distances and directions can be taken. For this purpose, consider the reference axis 82 to be oriented generally perpendicular to the anterior surface 80 of cornea 18, and to intersect the anterior surface 80 at a start point 84. The comeal incisions are made using a laser light beam.

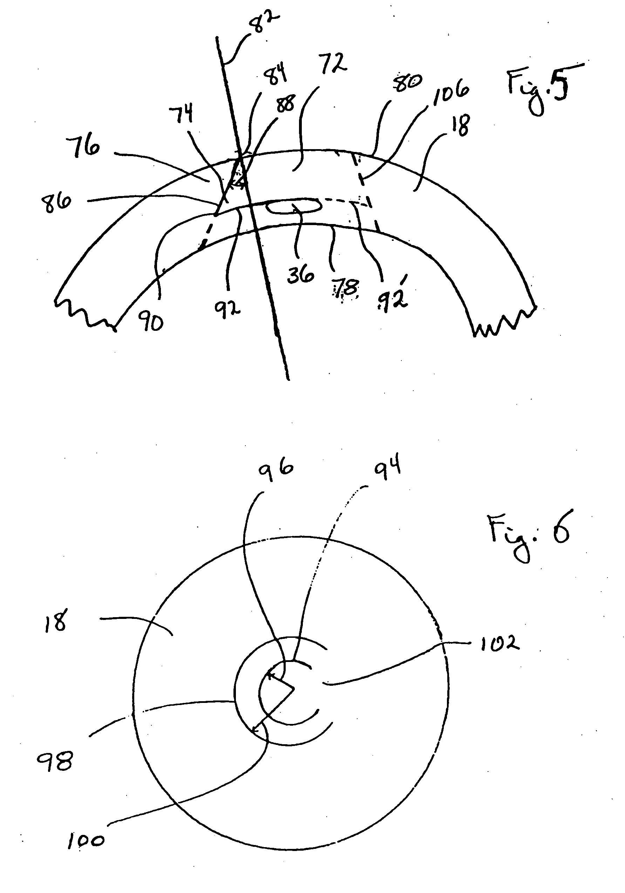

The creation of layer 72 begins by cutting the corneal tissue along a path 86 which is oriented at an angle 88 from the reference axis 82, and which extends from the start point 84 to a turn point 90. In practice, depending upon the particular desires of the operator, the path 86 can result in several different configurations for layer 72. One configuration, of course, is as shown in Figure 5. For this particular configuration, the path 86 is essentially a straight line. In this case, the straight line is set at an acute angle 88 relative to the reference axis 82, and it extends from the start point 84 to the turn point 90. The undercut region 74 is then formed by cutting back toward the reference axis 82 along a path 92 which generally parallels the posterior surface 78. Again, depending on the desires of the operation, the path 86 can be something other than a straight line, so long as the start point 88 is generally located on the reference axis 82 and the turn point 90 is distanced from the axis 82 to create the undercut region 74.

-The implication from the above disclosure is that a plurality of different cuts, each along the different paths 86 and 92, need to be made in order to create the layer 72. Reference to Figure 6, shows what the result of these several cuts might be. In Figure 6 it will be seen that a plurality of start points 84 can be selected to establish a periphery 94. As shown, the periphery 94 is curvilinear and can be generally defined by a radius of curvature 96. Further, it can be appreciated by reference to Figure 6 that a plurality of turn points 90 can be selected to establish a periphery 98. As shown, the periphery 98 is also curvilinear and can be generally defined by a radius of curvature 100. In order for the undercut region 74 to be established, it will be understood that the radius of curvature 100 must be greater than the radius of curvature 96. Additionally, as shown in Figure 6, the peripheries 94 and 98 do not close. Thus, the hinge area 102 is created and the layer 72 can be lifted as a flap in rotation about the hinge 102.

Once the flap 72 has been created, it can be mechanically lifted to expose an underlying capsule volume 36. As indicated above, the volume 36 can have many different sizes and shapes depending on the particular optical problem being confronted. In any case, once exposed, the volume 36 can be removed by procedures well known in the pertinent art. Importantly, after the volume 36 is removed, the layer 72 can be repositioned. When so repositioned, it is intended that the undercut region 74 will interact with overlap region 76 to restrain any further movement of the layer 72 in an anterior direction.

## Claims

1. A computer controlled laser surgical apparatus (10) adapted and programmed to create automatically a lentoid volume (36) adjacent a second surface area more posterior in the cornea than the anterior surface of the cornea;
said apparatus being further adapted and programmed to create automatically an interlocking flap (22) of corneal tissue by;
establishing a first periphery (54) having a first surface area on the anterior surface of the cornea;
establishing a second periphery (98) of the second surface area, said second surface area being larger than said first surface area and being substantially parallel thereto;
cutting between said first periphery and said second periphery; and
back cutting along said second surface to create an undercut region for said flap with a hinge between said flap and said cornea;
said undercut region restraining movement of said flap in a direction toward the anterior surface of the cornea during subsequent healing between said flap and the cornea.

2. Laser apparatus as claimed in claim 1, wherein forming a lentoid volume comprises cutting along a posterior surface of the lentoid volume; and
back cutting along an anterior surface of the lentoid volume so as to encapsulate and allow removal of the encapsulated lentoid volume from the cornea.

3. Laser apparatus as claimed in claim 1, wherein the lentoid volume comprises a convex anterior surface and removal of corneal tissue from the lentoid volume effects correction of myopia.

4. Laser apparatus as claimed in claim 1, wherein the lentoid volume comprises a concave posterior surface and removal of corneal tissue from the lentoid volume effects correction of hyperopia.

## Patentansprüche

1. Computergesteuerte Laserchirurgie-Vorrichtung (10), die dazu eingerichtet und programmiert ist, automatisch ein Linsenvolumen (36) nahe eines zweiten Oberflächenbereichs weiter hinten in der Hornhaut als die vordere Oberfläche der Hornhaut zu erstellen;
wobei die Vorrichtung ferner dazu eingerichtet und programmiert ist, automatisch einen formschlüssigen Lappen (72) aus Hornhautgewebe zu erzeugen, durch:
Bestimmen eines ersten Umfangs (94) mit einem ersten Oberflächenbereich auf der vorderen Oberfläche der Hornhaut;
Bestimmen eines zweiten Umfangs (98) des zweiten Oberflächenbereichs, wobei der zweite Oberflächenbereich größer als der erste Oberttächenbereich und im Wesentlichen parallel dazu ist;
Ausführen eines Schnittes zwischen dem ersten Umfang und dem zweiten Umfang; und
Zurückschneiden entlang der zweiten Oberfläche, so dass eine hinterschnittene Region für den Lappen mit einem Band zwischen dem Lappen und der Hornhaut entsteht;
wobei die hinterschnittene Region eine Bewegung des Lappens in einer Richtung zur vorderen Oberfläche der Hornhaut während des nachfolgenden Verheilungsprozesses zwischen Lappen und Hornhaut verhindert.

2. Laser-Vorrichtung nach Anspruch 1, wobei das Bilden eines Linsenvolumens das Schneiden entlang einer hinteren Oberfläche des Linsenvolumens umfasst; und das Zurückschneiden entlang einer vorderen Oberfläche des Linsenvolumens, um es einzukapsein und das Entfernen des eingekapselten Linsenvolumens von der Hornhaut zu ermöglichen.

3. Laser-Vorrichtung nach Anspruch 1, wobei das Linsenvolumen eine konvexe vordere Oberfläche umfasst und das Entfernen von Hornhautgewebe von dem Linsenvolumen die Korrektur von Kurzsichtigkeit bewirkt.

4. Laser-Vorrichtung nach Anspruch 1, wobei das Linsenvolumen eine konkave hintere Oberfläche umfasst und das Entfernen von Hornhautgewebe von dem Linsenvolumen die Korrektur von Weitsichtigkeit bewirkt.

## Revendications

1. Appareil chirurgical laser commandé par ordinateur (10) adapté et programmé pour créer automatiquement un volume lentoïde (36) adjacent à une seconde surface région plus en arrière dans la cornée que la surface antérieure de la cornée ;
ledit appareil étant en outre adapté et programmé pour créer automatiquement un lambeau de verrouillage (72) du tissu cornéen en :
établissant une première périphérie (54) ayant une première surface région sur la surface antérieure de la cornée ;
établissant une seconde périphérie (98) de la seconde surface région, ladite seconde surface région étant plus grande que ladite première surface région et étant sensiblement parallèle à celle-ci ;
coupant entre ladite première périphérie et ladite seconde périphérie ; et
recoupant le long de ladite seconde surface pour créer une région dégagée pour ledit lambeau avec une articulation entre ledit lambeau et ladite cornée ;
ladite région dégagée retenant le mouvement dudit lambeau dans une direction vers la surface antérieure de la cornée pendant la cicatrisation ultérieure entre ledit lambeau et la cornée.

2. Appareil laser selon la revendication 1, dans lequel l'étape consistant à former un volume lentoïde comprend l'étape consistant à couper le long d'une surface postérieure du volume lentoïde ; et
l'étape consistant à recouper le long d'une surface antérieure du volume lentoïde afin d'encapsuler et de permettre le retrait du volume lentoïde encapsulé de la cornée.

3. Appareil laser selon la revendication 1, dans lequel le volume lentoïde comprend une surface antérieure convexe et le retrait du tissu cornéen du volume lentoïde réalise la correction de la myopie.

4. Appareil laser selon la revendication 1, dans lequel le volume lentoïde comprend une surface postérieure concave et le retrait du tissu cornéen du volume lentoïde réalise la correction de l'hypermétropie.
